# EUROPEAN PATENT APPLICATION

(11) **EP 4 667 628 A1**
(43) Date of publication of application: **24.12.2025**
(21) Application number: 24183190.8
(22) Date of filing: 19.06.2024
(51) Int. Cl.: C40B 30/06, G01N 33/50

(54) **SYNTHETIC LETHALITY TO TREAT AUTOSOMAL DOMINANT POLYCYSTIC KIDNEY DISEASE (ADPKD)**

(71) Applicant: Albert-Ludwigs-Universität Freiburg Körperschaft des öffentlichen Rechts, 79098 Freiburg (DE)
(72) Inventor: Köttgen, Michael, 79100 Freiburg (DE); Westermann, Lukas, 79102 Freiburg (DE)
(74) Representative: Kraus & Lederer PartGmbB

(57) **Abstract**

The present application relates to a process for identifying a compound useful for treating autosomal dominant polycystic kidney disease (ADPKD) and compounds for use in the treatment of ADPKD.

## Description

### Background of the invention

The present invention relates (1) an assay for cell-based high-throughput screening for synthetic lethality in autosomal dominant polycystic kidney disease and (2) compounds for use in the treatment of autosomal dominant polycystic kidney disease.

Kidney disease affects 10-15% of adults worldwide and is a leading cause of death. Chronic kidney disease substantially increases the risk of cardiovascular mortality.' Autosomal Dominant Polycystic Kidney Disease (ADPKD) is the most common monogenic cause of end-stage kidney disease (ESKD) and affects 12 million patients worldwide.² The 5-year probability of survival among ADPKD patients with ESKD is less than 40%.³ There is no approved therapy to cure ADPKD, and current treatments include dialysis and transplantation, which are burdensome and costly. Here, a novel therapeutic approach for the treatment of ADPKD is proposed based on the concept of synthetic lethality.

ADPKD is caused by mutations in *PKD1* or *PKD2.*² Cyst formation results from loss of heterozygosity through somatic mutations in kidney tubular epithelial cells.^{4,5} Cysts form only in a small fraction of nephrons (1-3%), while the heterozygous tubular epithelial cells retain their normal form and function. Initially, cysts are microscopically small and slowly grow over decades through proliferation and secretion. Mechanical compression of healthy kidney tissue causes functional decline and eventually leads to ESKD. Current experimental therapies and the only approved treatment of ADPKD, Tolvaptan, target cyst growth by slowing proliferation and secretion.⁶ However, the efficacy of these approaches is rather modest and there are considerable side effects.

Thus, there is a need to identify compounds that can be used to treat ADPKD. In addition, there is a need to provide an assay for identifying such compounds.

### Summary of the invention

The present invention relates a process for identifying a compound useful for treating autosomal dominant polycystic kidney disease (ADPKD), the process comprising:
(a) contacting a test compound with PKD1-deficient and/or PKD2-deficient cells;
(b) determining cell viability of the PKD1-deficient and/or PKD2-deficient cells; and
(c) identifying useful compounds by confirming that cell viability of the PKD1-deficient and/or PKD2-deficient cells is lower than that of isogenic control cells.

The present invention aims at applying the concept of synthetic lethality⁷ to the treatment of ADPKD. Synthetic lethality will allow to selectively kill homozygous mutant cyst epithelial cells, whereas heterozygous cells survive. This strategy can reduce cyst burden with moderate side effect as a drug based on this concept can be applied for limited time periods and may not require constant treatment over decades.

In addition, the present invention relates to a compound for use in the treatment of autosomal dominant polycystic kidney disease (ADPKD),
wherein the compound is selected from a compound formula (I): 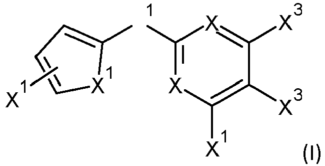 wherein
R¹ is selected from nitro, cyano, halo, alkoxy, alkyl and carboxy,
X¹ is selected from NH, N(alkyl), and O,
L¹ is a linker group selected from C₁-C₆ alkylene, C₂-C₆ alkenylene, NHC(O), C(O)NH, S(O)₂NH, NHS(O)₂, NHC(O)NH, and S(O)₂,
X² is selected from CR^{0a}, and N,
X³ is selected from CR^{0c}, and N,
R^{0a}, R^{0c}, and R¹ are independently selected from hydroxy, halo, hydrogen, alkyl, haloalkyl, alkoxy, haloalkoxy, sulfamoyl, sulfamoylalkyl, N,N-(dialkyl)-sulfamoyl, and N-alkylsulfamoyl,
R² and R³ form, together with the carbon atoms to which they are attached, an optionally substituted 5- or 6-membered aromatic group, or R² and R³ are independently selected from halo, hydrogen, alkyl, haloalkyl, alkoxy, haloalkoxy, sulfamoyl, sulfamoylalkyl, N,N-(dialkyl)-sulfamoyl, and N-alkylsulfamoyl;
a compound of formula (II): 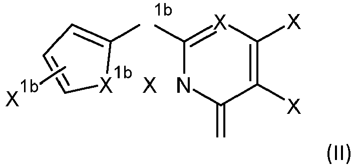 wherein
   R^{1b} is selected from nitro, cyano, halo, alkoxy, alkyl and carboxy,
   X^{1b} is selected from NH, N(alkyl), and O,
   L^{1b} is a linker group selected from C₁-C₆ alkylene, C₂-C₆ alkenylene, NHC(O), C(O)NH, S(O)₂NH, NHS(O)₂, NHC(O)NH, and S(O)₂,
   X^{2b} is CR^{1b} or N,
   R^{0b} and R^{1b} are independently selected from optionally substituted 5- or 6-membered aromatic group, hydroxy, halo, hydrogen, alkyl, haloalkyl, alkoxy, haloalkoxy, sulfamoyl, sulfamoylalkyl, N,N-(dialkyl)-sulfamoyl, and N-alkylsulfamoyl,
   R^{2b} and R^{3b} form, together with the carbon atoms to which they are attached, an optionally substituted 5- or 6-membered aromatic group, or R^{2b} and R^{3b} are independently selected from halo, hydrogen, alkyl, haloalkyl, alkoxy, haloalkoxy, sulfamoyl, sulfamoylalkyl, N,N-(dialkyl)-sulfamoyl, and N-alkylsulfamoyl;
   a compound of formula (III): 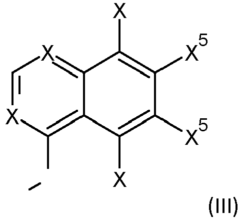 wherein
      X⁴ and X⁵ are independently selected from CH and N,
      L² is NH, O, or S(O)₂,
      L³ is an optionally substituted 5- or 6-membered aromatic group, and
      R⁴ to R⁷ are independently selected from H, halo, nitro, cyano, optionally substituted alkoxy, alkyl, and haloalkyl;
      MEK inhibitors, 1-methyl-3-[(Z)-(4-oxo-3-prop-2-enyl-1,3-thiazolidin-2-ylidene)amino]thiourea, 2-TEDC, CCCP, 5-nitro-N-{2-[(2-pyridinylmethyl)carbamoyl]phenyl}-2-furamide, N-benzyl-6-phenyl-2-pyridin-2-ylpyrimidin-4-amine, 6-(2-fluorophenyl)-N-[2-(1H-indol-3-yl)ethyl]-2-(2-pyridinyl)-4-pyrimidinamine, 4-piperidin-1-yl-6-propan-2-yl-2-pyridin-2-ylpyrimidine, pterocarpanquinone, 5-chloro-4-methyl-2-(2-pyridinyl)-6-(1-pyrrolidinyl)pyrimidine, GSK-J4, N-(4-{[6-(cyclobutylamino)-7H-purin-2-yl]amino}phenyl)-N-methylacetamide, 5-(4-methoxybenzyl)-2-(2-pyridinyl)-4,6-pyrimidinediamine, dasatinib, dimorpholinethiuram disulfide, FCCP, and PD184352; as well as pharmaceutically acceptable salts, solvates, polymorphs, enantiomers, and tautomers thereof.

These compounds have been identified by the above-mentioned process.

### Detailed description

### Definitions

The following definitions are relevant in connection with the embodiments of the present invention.

The prefix "C_{y}-Cₓ" as used herein refers to the number of carbon atoms of the respective group.

As used herein, singular forms, such as "a", "an", and "the", include both singular and plural referents unless the context clearly dictates otherwise.

The meaning of the term "comprising" is to be interpreted as encompassing all the specifically mentioned features as well optional, additional, unspecified ones, whereas the term "consisting of" only includes those features as specified. Therefore, "comprising" includes as a limiting case the composition specified by "consisting of".

The term "PKD1-deficient cells" refers to cells that comprise a genetically modified disruption in the PKD1 gene, which results in deficiency in expression and/or function. This can be achieved *inter alia* by gene mutation or gene silencing. PKD1-deficient cells encompass cells that comprise a PKD1^{-/-} genetic background in all copies of the PKD1 gene (referred to as PKD1^{-/-} cells). Thus, the term PKD1-deficient cells includes PKD1 knockout and PKD1 knockdown cells. Analogous considerations apply for the term "PKD2-deficient cells".

The term "test compound" refers to a compound that is being tested. Such test compounds can be provided as part of compound libraries.

The term "5-membered aromatic group" as used herein by itself or as part of another group refers to an aromatic ring system having 1 to 3 three heteroatoms, such as N, O, or S. Non-limiting exemplary 5-membered aromatic groups include furyl, pyrrolyl, pyrazolyl, imidazolyl, triazolyl, oxazolyl, thienyl, and pyridyl.

The term "6-membered aromatic group" as used herein encompasses 6-membered heteroaryl groups, such as pyridyl, pyridazinyl, pyrimidinyl, pyrazinyl, or 1,2,3-triazinyl, and 6-membered homoaryl groups, such as phenyl (abbreviated as "Ph").

The term "optionally substituted" 5- or 6-membered aromatic groups refers to a 5- or 6-membered ring that is optionally substituted by one or more, such as two, three or four, substituents independently selected from hydroxy, halo, alkyl, haloalkyl, alkoxy, haloalkoxy, sulfamoyl, nitro and cyano.

The term "alkyl" as used herein by itself or as part of another group refers to a straight- or branched-chain aliphatic hydrocarbon containing one to twelve carbon atoms (i.e., C₁-C₁₂ alkyl). In one embodiment, the alkyl group is chosen from a straight chain C₁-C₁₀ alkyl group. In another embodiment, the alkyl group is chosen from a branched chain C₃-C₁₀ alkyl group. In another embodiment, the alkyl group is chosen from a straight chain C₁-C₆ alkyl group. In another embodiment, the alkyl group is chosen from a branched chain C₃-C₆ alkyl group. In another embodiment, the alkyl group is chosen from a straight chain C₁-C₄ alkyl group. In another embodiment, the alkyl group is chosen from a branched chain C₃-C₄ alkyl group. In another embodiment, the alkyl group is chosen from a straight or branched chain C₃-C₄ alkyl group. Non-limiting exemplary C₁-C₁₀ alkyl groups include methyl, ethyl, propyl, isopropyl, butyl, sec-butyl, tert-butyl, iso-butyl, 3-pentyl, hexyl, heptyl, octyl, nonyl, decyl, and the like. Non-limiting exemplary C₁-C₄ alkyl groups include methyl, ethyl, propyl, isopropyl, butyl, sec-butyl, tert-butyl, and iso-butyl.

The skilled person will understand that the term "alkyl" as used herein also encompasses "alkylene", including as non-limiting examples e.g. methylene (-CH₂-), ethylene (-CH₂CH₂-), or propylene (-CH₂CH₂CH₂-), when the alkyl group is not a terminal group, but is part of a chain. Similar considerations apply for other functional groups.

The term "alkenyl" as used herein by itself or as part of another group refers to an alkyl group as defined above containing one, two or three carbon-to-carbon double bonds. In one embodiment, the alkenyl group is chosen from a C₂-C₆ alkenyl group having 2, 3, 4, 5 or 6 carbon atoms. In another embodiment, the alkenyl group is chosen from a C₂-C₄ alkenyl group having 2, 3, or 4 carbon atoms. Non-limiting exemplary alkenyl groups include ethenyl, propenyl, isopropenyl, butenyl, sec-butenyl, pentenyl, and hexenyl.

The term "alkoxy" as used herein by itself or as part of another group refers to an optionally substituted alkyl, optionally substituted alkenyl or optionally substituted alkynyl attached to a terminal oxygen atom. In one embodiment, the alkoxy group is chosen from a C₁-C₆ alkoxy group. In another embodiment, the alkoxy group is chosen from a C₁-C₄ alkyl attached to a terminal oxygen atom, e.g., methoxy, ethoxy, tert-butoxy, pentoxy and hexoxy. It is preferred that the term "alkoxy" refers to a linear C₁-C₆ alkyl group attached to a terminal oxygen atom.

The term "haloalkyl" as used herein by itself or as part of another group refers to an alkyl that is substituted with at least on halo group. Non-limiting examples of haloalkyl groups are CF₃, CH₂F, and CHF₂. The term "haloalkoxy" as used herein by itself or as part of another group refers to an alkoxy group that is substituted with at least on halo group. Non-limiting examples of haloalkoxyl groups are OCF₃, OCH₂F, and OCHF₂, OCF₂CF₃, OCH₂CF₃, and OCH₂CH₂F.

The term "halo" encompasses F, Cl, Br, and I.

Composite terms as used herein, such as "sulfamoylalkyl", are to be understood in their broadest technically sensible way and may refer to a connectivity of -sulfamoylalkyl or sulfamoylalkyl-. However, it is preferred that composite terms are interpreted in that the last-mentioned group is connected "first", e.g. -alkyl-SO₂NH. In case the composite term has more than one bond/connection, it is preferred that the last-mentioned group is connected first and the first mentioned group connects the group to the next substituent.

The term "sulfamoyl" as used herein by itself or as part of another group refers to a -SO₂NH₂ group.

The term "*N,N*-(dialkyl)-sulfamoyl" refers to a -SO₂-N(alkyl)₂ group. The term "N-alkylsulfamoyl" refers to a -SO₂-NH(alkyl) group

The term "subject" refers to human and non-human animals, including all vertebrates, e.g., mammals and non-mammals, such as non-human primates, mice, rabbits, sheep, dogs, cats, horses, cows, chickens, amphibians, and reptiles. In most particular embodiments of the described methods, the subject is a human.

The terms "treating" or "treatment" refer to any success or indicia of success in the attenuation or amelioration of the disease, including any objective or subjective parameter such as abatement, remission, diminishing of symptoms or making the condition more tolerable to the patient, slowing in the rate of degeneration or decline, making the final point of degeneration less debilitating, improving a subject's physical or mental well-being, or prolonging the length of survival. The treatment may be assessed by objective or subjective parameters; including the results of a physical examination.

An "effective amount"" refers to an amount effective, at dosages and for periods of may vary according to factors such as the disease state, age, sex, and weight of the individual, and the ability of the compound to elicit a desired response in the individual. A therapeutically effective amount is also one in which any toxic or detrimental effects of the therapeutic are outweighed by the therapeutically beneficial effects.

The term "pharmaceutically acceptable salts" refers to - in case the compound has a basic functional group - the addition salt of the free base with hydrochloric acid, hydrobromic acid, boric acid, phosphoric acid, acetic acid, citric acid, fumaric acid, maleic acid, malic acid, malonic acid, oxalic acid, succinic acid, tartaric acid, p-toluenesulfonic acid, trifluoromethanesulfonic acid, or trifluoroacetic acid. In case the compound has an acidic functional group, the term "pharmaceutically acceptable salt" refers to the addition salt of the acid with an alkaline metal salt such as a sodium salt, potassium salt, cesium salt; alkaline earth metals such as a magnesium salt, calcium salt, and ammonium salts. sodium salt, potassium salt, cesium salt, and the like; alkaline earth metals such as calcium salt, magnesium salt and the like; organic amine salts such as triethylamine salt, pyridine salt, picoline salt, ethanolamine salt, triethanolamine salt, dicylohexylamine salt, N,N'-dibenzylethylenediamine salt.

The term "solvate" refers to the compound complexed with a solvent molecule (including water) that is bond by intermolecular forces in the crystal lattice of the compound.

Preferred embodiments according to the invention are defined hereinafter. The preferred embodiments are preferred alone or in combination. Further, it is to be understood that the following preferred embodiments refer to all aspects of the present invention, i.e. the process, and the compound for use.

### The process

In an embodiment, the present invention relates to a process for identifying a compound useful for treating autosomal dominant polycystic kidney disease (ADPKD), the process comprising:
(a) contacting a test compound with PKD1-deficient and/or PKD2-deficient cells;
(b) determining cell viability of the PKD1-deficient and/or PKD2-deficient cells; and
(c) identifying useful compounds by confirming that cell viability of the PKD1-deficient and/or PKD2-deficient cells is lower than that of isogenic control cells.

It is preferred that the cells are PKD1-deficient cells. Thus, in a preferred embodiment, the process comprises:
(a) contacting a test compound with PKD1-deficient cells;
(b) determining cell viability of the PKD1-deficient cells; and
(c) identifying useful compounds by confirming that cell viability of the PKD1-deficient cells is lower than that of isogenic control cells.

The rationale for the present invention is the fact that cyst number and volume are the main determinants of disease severity in ADPKD.⁸ Selective killing of cyst-lining epithelial cells, harboring homozygous *PKD1* for *PKD2* mutations), promises to reduce cyst number and size while sparing phenotypically normal heterozygous cells. The concept of synthetic lethality has been validated clinically through the demonstrated efficacy of poly(ADP-ribose) polymerase (PARP) inhibitors for the treatment of cancers in individuals with germline loss-of-function mutations in either *BRCA1* or *BRCA2.*⁹

The present invention relates to the concept of synthetic lethality for the application in ADPKD by conducting a chemical synthetic lethality screen in *PKD1*-deficient mIMCD-3 cells and isogenic control cells. The screen focused on *PKD1* since mutations in this gene account for the majority (∼80%) of ADPKD cases in humans. Cytotoxicity of compounds in wildtype and homozygous mutant cells.

The translation of findings from preclinical models to clinical trials investigating synthetic lethality as a therapeutic approach for ADPKD is feasible. Interventional clinical trials at early stages of kidney diseases have been challenging in the past due to the difficult selection of appropriate primary outcomes.¹⁰ For ADPKD, this has recently changed and the approval of Tolvaptan has paved the way to translation.⁶ The prospective follow-up of ADPKD patients with magnetic resonance imaging (MRI) examinations in the CRISP cohort has established that cysts increase at a steady rate of around 5% per year, and that total kidney volume (TKV) is the strongest predictor of kidney function decline in ADPKD.^{8,11} TKV is globally considered a useful marker for progression in ADPKD.¹² In terms of clinical trials, regulatory agencies have now accepted TKV as a prognostic biomarker and surrogate endpoint, and as a basis for accelerated approval.

It has been found that the assay of the present invention is robust and a well-controlled high-throughput assay for the identification of chemical compounds that result in synthetic lethality in cyst epithelial cells of ADPKD patients thus reducing cyst number and size. It has been found that the assay of the present invention can be used to screen large compound libraries with excellent assay quality. In addition, it has been found that by using isogenic control cells, false-positive hits can be minimized.

It is preferred that the cell viability of the PKD1-deficient cells and the viability of isogenic control cells is determined under identical conditions.

The term "useful" refers a compound that potentially can treat ADPKD, and preferably to a compound that is effective in the treatment of ADPKD. The process of the present invention is an in-vitro process.

In an embodiment, the invention relates to a process for identifying a compound for use in the treatment of autosomal dominant polycystic kidney disease (ADPKD), the process comprising:
(a) contacting a test compound with PKD1-deficient cells;
(b) determining cell viability of the PKD1-deficient cells; and
(c) identifying compounds by confirming that cell viability of the PKD1-deficient cells is lower than that of isogenic control cells.

In an embodiment, the cell viability is measured by a cell viability assay, preferably a cell counting assay, a terminal deoxynucleotidyl transferase nick end labeling assay, an immunohistochemical assay, or a ATP-dependent assay. It is particularly preferred that the cell viability is measured with a ATP-dependent assay. Non-limiting examples of such an assay is the luminescence-based CellTiter-Glo^{®} 2.0 assay. In the examples, the assay was performed in *PKD1*-deficient cells with isogenic control cells using a luminescence-based ATP-dependent assay for cell viability (CellTiter-Glo^{®} 2.0). Compounds were applied at final concentrations of 10 µM. Hits from the primary screen qualified for comprehensive dose-response analyses with simultaneous isogenic wildtype counter screens. Cell viability in *PKD1*-deficient cells were compared to corresponding wildtype viability by determining IC₅₀, i.e. the concentration that kills 50% of the cells. Compounds with lower IC₅₀ values in *PKD1* knockout cells compared to isogenic control cells were investigated in more detail. In this screen, multiple compounds that kill *PKD1*-deficient cells more efficiently than wildtype cells were identified.

In an embodiment, the PKD1-deficient cells are homozygous cells, preferably PKD1 knockout cells. The PKD1-deficient cells are homozygous cells.

In a particularly preferred embodiment, the process of the present invention comprises the steps of:
(a) contacting a test compound with PKD1 knockout cells;
(b) determining cell viability of the PKD1 knockout cells;
(c) identifying useful compounds by confirming that viability of the PKD1-deficient cells is lower than that of isogenic control cells; and
(d) confirming the results with PKD1 knockdown cells.

The step (d) may comprise the sub-steps of:
(da) contacting a test compound and/or a compound identified in step (c) with PKD1 knockdown cells;
(db) determining cell viability of the PKD1 knockdown cells;
(dc) identifying useful compounds by confirming that cell viability of the PKD1 knockdown cells is lower than that of isogenic control cells.

Preferably, the PKD1-deficient and/or PKD2-deficient cells are obtainable by genome editing of cells, preferably mIMCD-3 LLC-PK1, RPTEC, HREpC or HK-2 cells. In an embodiment, the cells are renal tubular cells. In another embodiment, the cells are from cell lines selected from C8161, CCRF-CEM, MOLT, mIMCD-3, LLC-PK1, RPTEC, HREpC, HK-2, NHDF, HeLa-S3, Huh1, Huh4, Huh7, HUVEC, HASMC, HEKn, HEKa, MiaPaCell, Panc1, PC-3, TF1, CTLL-2, CIR, Rat6, CV1, RPTE, A10, T24, J82, A375, ARH-77, Calu1, SW480, SW620, SKOV3, SK-UT, CaCo2, P388D1, SEM-K2, WEHI-231, HB56, TIB55, Jurkat, J45.01, LRMB, Bcl-1, BC-3, IC21, DLD2, Raw264.7, NRK, NRK-52E, MRC5, MEF, Hep G2, HeLa B, HeLa T4, COS, COS-1, COS-6, COS-M6A, BS-C-1 monkey kidney epithelial, BALB/3T3 mouse embryo fibroblast, 3T3 Swiss, 3T3-L1, 132-d5 human fetal fibroblasts; 10.1 mouse fibroblasts, 293-T, 3T3, 721, 9L, A2780, A2780ADR, A2780cis, A172, A20, A253, A431, A-549, ALC, B16, B35, BCP-1 cells, BEAS-2B, bEnd.3, BHK-21, BR 293, BxPC3, C3H-10T1/2, C6/36, Cal-27, CHO, CHO-7, CHO-IR, CHO-K1, CHO-K2, CHO-T, CHO Dhfr-/-, COR-L23, COR-L23/CPR, COR-L23/5010, COR-L23/R23, COS-7, COV-434, CML T1, CMT, CT26, D17, DH82, DU145, DuCaP, EL4, EM2, EM3, EMT6/AR1, EMT6/AR10.0, FM3, H1299, H69, HB54, HB55, HCA2, HEK-293, HeLa, Hepa1c1c7, HL-60, HMEC, HT-29, Jurkat, JY cells, K562 cells, Ku812, KCL22, KG1, KYO1, LNCap, Ma-Mel 1-48, MC-38, MCF-7, MCF-10A, MDA-MB-231, MDA-MB-468, MDA-MB-435, MDCK II, MOR/0.2R, MONO-MAC 6, MTD-1A, MyEnd, NCI-H69/CPR, NCI-H69/LX10, NCI-H69/LX20, NCI-H69/LX4, NIH-3T3, NALM-1, NW-145, OPCN/OPCT cell lines, Peer, PNT-1A/PNT 2, RenCa, RIN-5F, RMA/RMAS, Saos-2 cells, Sf-9, SkBr3, T2, T-47D, T84, THP1 cell line, U373, U87, U937, VCaP, Vero cells, WM39, WT-49, X63, YAC-1, YAR, and transgenic varieties thereof.

In a preferred embodiment, the PKD1-deficient and/or PKD2-deficient cells are obtainable by using a CR!SPR-Cas nickase system, preferably a CR!SPR-Cas9 system. The CRISPR-Cas system is generally described by J. Doudna and E. Charpentier in EP 2 800 811. The CRISPR-Cas 9 system comprises a Cas9 polypeptide, and a single-molecule DNA-targeting RNA (also referred to as guide RNA) comprising (i) a DNA-targeting segment comprising a nucleotide sequence that is complementary to a sequence in the target DNA, and (ii) a protein-binding segment that interacts with said Cas9 polypeptide, wherein the protein-binding segment comprises two complementary stretches of nucleotides that hybridize to form a double stranded RNA (dsRNA) duplex. It is preferred monoclonal PKD1 KO cell lines are generated and compared with isogenic control cells. Specifically, it is preferred that the CRISPR-Cas system described in the literature^{14, 15} is used, comprising a nickase-expressing plasmid pX335-U6-Chimeric-BB-CBh-hSpCas9n (Addgene, cat. no. 42335) with two different gRNAs to induce two double strand breaks resulting in a PKD1 null allele.

In an embodiment, the compound useful for treating ADPKD has a ratio of IC₅₀ for PKD1-deficient cells to IC₅₀ for isogenic control cells of less than 0.85, less than 0.90, less than 0.85 or less than 0.80. The term IC₅₀ refers to the concentration of the compound that kills 50% of the cells.

In an embodiment, contacting in step (a) takes place at about 37°C for at least 12 hours, at least 24 hours, at least 36 hours, at least 48 hours, or at least 60 hours. Preferably, contacting in step (a) takes place in an incubator, more preferably at a 5 % CO₂ level.

The steps of the process of the present invention allow to provide a high-throughput screening assay (also referred to as HTS assay). This HTS assay allows to efficiently screen large compound libraries. It is preferred that the contacting step takes place on well plates, such as 96-, 384-, or 1536-well plates. Preferably, the well plates comprise basins that contain water. This allows to remove edge-effects due to excessive evaporation of water from the well plates.

### Compound for use

The present invention relates to compound for use in the treatment of autosomal dominant polycystic kidney disease (ADPKD),
wherein the compound is selected from a compound formula (I): 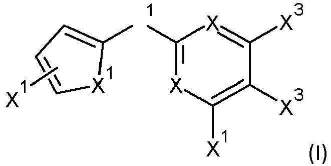 wherein
R¹ is selected from nitro, cyano, halo, alkoxy, alkyl and carboxy,
X¹ is selected from NH, N(alkyl), and O;
L¹ is a linker group selected from C₁-C₆ alkylene, C₂-C₆ alkenylene, NHC(O), C(O)NH, S(O)₂NH, NHS(O)₂, NHC(O)NH, and S(O)₂,
X² is selected from CR^{0a}, and N,
X³ is selected from CR^{0c}, and N,
R^{0a}, R^{0c}, and R¹ are independently selected from hydroxy, halo, hydrogen, alkyl, haloalkyl, alkoxy, haloalkoxy, sulfamoyl, sulfamoylalkyl, N,N-(dialkyl)-sulfamoyl, and N-alkylsulfamoyl,
R² and R³ form, together with the carbon atoms to which they are attached, an optionally substituted 5- or 6-membered aromatic group, or R² and R³ are independently selected from halo, hydrogen, alkyl, haloalkyl, alkoxy, haloalkoxy, sulfamoyl, sulfamoylalkyl, N,N-(dialkyl)-sulfamoyl, and N-alkylsulfamoyl;
a compound of formula (II): 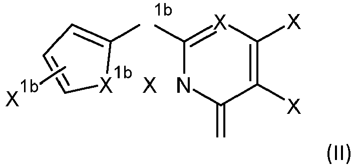 wherein
   R^{1b} is selected from nitro, cyano, halo, alkoxy, alkyl and carboxy,
   X^{1b} is selected from NH, N(alkyl), and O;
   L^{1b} is a linker group selected from C₁-C₆ alkylene, C₂-C₆ alkenylene, NHC(O), C(O)NH, S(O)₂NH, NHS(O)₂, NHC(O)NH, and S(O)₂,
   X^{2b} is CR^{1b} or N,
   R^{0b} and R^{1b} are independently selected from optionally substituted 5- or 6-membered aromatic group, hydroxy, halo, hydrogen, alkyl, haloalkyl, alkoxy, haloalkoxy, sulfamoyl, sulfamoylalkyl, N,N-(dialkyl)-sulfamoyl, and N-alkylsulfamoyl,
   R^{2b} and R^{3b} form, together with the carbon atoms to which they are attached, an optionally substituted 5- or 6-membered aromatic group, or R^{2b} and R^{3b} are independently selected from halo, hydrogen, alkyl, haloalkyl, alkoxy, haloalkoxy, sulfamoyl, sulfamoylalkyl, N,N-(dialkyl)-sulfamoyl, and N-alkylsulfamoyl;
   a compound of formula (III): 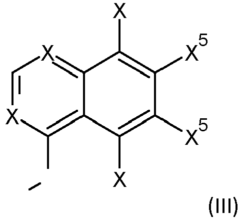 wherein
      X⁴ and X⁵ are independently selected from CH and N,
      L² is NH, O, or S(O)₂;
      L³ is an optionally substituted 5- or 6-membered aromatic group, and
      R⁴ to R⁷ are independently selected from H, halo, nitro, cyano, optionally substituted alkoxy, alkyl, and haloalkyl;
      MEK inhibitors, 1-methyl-3-[(Z)-(4-oxo-3-prop-2-enyl-1,3-thiazolidin-2-ylidene)amino]thiourea, 2-TEDC, CCCP, 5-nitro-N-{2-[(2-pyridinylmethyl)carbamoyl]phenyl}-2-furamide, N-benzyl-6-phenyl-2-pyridin-2-ylpyrimidin-4-amine, 6-(2-fluorophenyl)-N-[2-(1H-indol-3-yl)ethyl]-2-(2-pyridinyl)-4-pyrimidinamine, 4-piperidin-1-yl-6-propan-2-yl-2-pyridin-2-ylpyrimidine, pterocarpanquinone, 5-chloro-4-methyl-2-(2-pyridinyl)-6-(1-pyrrolidinyl)pyrimidine, GSK-J4, N-(4-{[6-(cyclobutylamino)-7H-purin-2-yl]amino}phenyl)-N-methylacetamide, 5-(4-Methoxybenzyl)-2-(2-pyridinyl)-4,6-pyrimidinediamine, dasatinib, dimorpholinethiuram disulfide, FCCP, and PD184352; as well as pharmaceutically acceptable salts, solvates, polymorphs, enantiomers, and tautomers thereof.

In an embodiment, the compound for use of the present invention is a compound of formula (I): 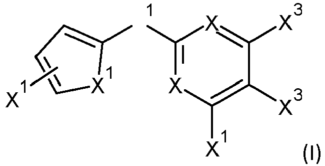 wherein
R¹ is selected from nitro, cyano, and halo,
X¹ is selected from NH, N(alkyl), and O,
L¹ is a linker group selected from NHC(O), C(O)NH, S(O)₂NH, NHS(O)₂, NHC(O)NH, and S(O)₂,
X² is CR^{0a},
X³ is selected from CR^{0c},
R^{0a}, R^{0c}, and R¹ are independently selected from hydroxy, halo, hydrogen, alkyl, haloalkyl, alkoxy, haloalkoxy, sulfamoyl, sulfamoylalkyl, N,N-(dialkyl)-sulfamoyl, and N-alkylsulfamoyl, and
R² and R³ are independently selected from halo, hydrogen, alkyl, haloalkyl, alkoxy, haloalkoxy, sulfamoyl, sulfamoylalkyl, N,N-(dialkyl)-sulfamoyl, and N-alkylsulfamoyl.

Preferably, R¹ is a nitro group.

X¹ is preferably O.

L¹ is preferably selected from NHC(O) and C(O)NH.

X² and X³ are CH.

R¹ is preferably selected from halo, hydrogen, alkyl, haloalkyl, alkoxy, and haloalkoxy.

R² and R³ are preferably independently selected from halo, hydrogen, alkyl, haloalkyl, alkoxy, haloalkoxy, sulfamoyl, *N,N*-(dialkyl)-sulfamoyl, and N-alkylsulfamoyl. Even more preferably R² is selected from trifluoromethyl, methoxy and *N,N*-(dimethyl)sulfamoyl. Even more preferably, R³ is selected from halo and hydrogen.

In a particularly preferred embodiment, the compound of formula (I) is N-[3-methoxy-5-(trifluoromethyl)phenyl]-5-nitro-2-furamide or N-[4-chloro-3-(dimethylsulfamoyl)phenyl]-5-nitro-2-furamide.

In an embodiment, the compound for use of the present invention is a compound of formula (II), 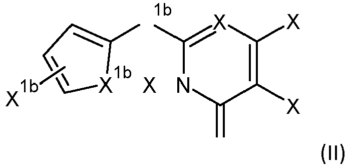 wherein
R^{1b} is selected from nitro, cyano, and halo,
X^{1b} is selected from NH, N(alkyl), and O;
L^{1b} is a linker group selected from C₁-C₆ alkylene and C₂-C₆ alkenylene,
X^{2b} is N,
R^{0b} is selected from optionally substituted 5- or 6-membered aromatic group and alkyl, and
R^{2b} and R^{3b} form, together with the carbon atoms to which they are attached, an optionally substituted 5- or 6-membered aromatic group.

It is preferred that R^{1b} is nitro.

It is preferred that X^{1b} is O.

It is preferred that L^{1b} is an ethenylene group, even more preferably a (Z)-ethenylene group.

It is preferred that R^{0b} is a C₁-C₆ alkyl or a substituted phenyl. It is particularly preferred that R^{0b} is methyl or phenyl that is substituted with one or more substituents independently selected from hydroxy, halo, alkyl and alkoxy.

It is preferred that R^{2b} and R^{3b} form, together with the carbon atoms to which they are attached, an optionally substituted 6-membered aromatic group. It is particularly preferred that R^{2b} and R^{3b} form, together with the carbon atoms to which they are attached, a phenyl group.

In an even more preferred embodiment, the compound of formula (II) is 3-(4-hydroxyphenyl)-2-[(E)-2-(5-nitrofuran-2-yl)ethenyl]quinazolin-4-one or 3-methyl-2-[(E)-2-(5-nitrofuran-2-yl)ethenyl]quinazolin-4-one

In an embodiment, the compound for use of the present invention is a compound of formula (III): 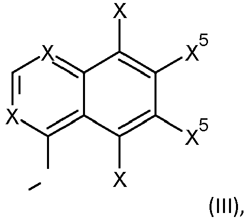 wherein
X⁴ and X⁵ are N,
L² is NH,
L³ is an optionally substituted phenyl group, preferably a disubstituted phenyl group, and
R⁴ to R⁷ are independently selected from H, halo, and alkoxy.

Preferably, R⁴ and R⁷ are hydrogen. Preferably, R⁵ and R⁶ are independently selected from H, halo, nitro, cyano, optionally substituted alkoxy, alkyl, and haloalkyl.

L³ is preferably a phenyl or substituted phenyl. More preferably, L³ is a phenyl group substituted with halo and/or alkyl, even more preferably a phenyl group that is substituted with a 3-halo-2-alykphenyl group.

In a particularly preferred embodiment, the compound of formula (III) is 7-chloro-N-(3-chloro-2-methylphenyl)-4-quinazolinamine, N-(3-chloro-2-methylphenyl)-6,7-dimethoxy-4-quinazolinamine, or N-(3-chloro-2-methylphenyl)-4-quinazolinamine.

In an embodiment, the compound for use of the present invention is a MEK inhibitor. Without being bound to theory, it has surprisingly been discovered that a significant amount of the compounds identified are MEK inhibitors. The MEK inhibitor may be selected from trametinib, cobimetinib, binimetinib, selumetinib, refametinib, mirdametinib, and Tak-733. In a preferred embodiment, the MEK inhibitor is selected from refametinib, mirdametinib, and Tak-733.

In a preferred embodiment, the compound for use is selected from 1-methyl-3-[(Z)-(4-oxo-3-prop-2-enyl-1,3-thiazolidin-2-ylidene)amino]thiourea, 2-TEDC, CCCP, 5-nitro-N-{2-[(2-pyridinylmethyl)carbamoyl]phenyl}-2-furamide, N-benzyl-6-phenyl-2-pyridin-2-ylpyrimidin-4-amine, 6-(2-fluorophenyl)-N-[2-(1H-indol-3-yl)ethyl]-2-(2-pyridinyl)-4-pyrimidinamine, 4-piperidin-1-yl-6-propan-2-yl-2-pyridin-2-ylpyrimidine, pterocarpanquinone, 5-chloro-4-methyl-2-(2-pyridinyl)-6-(1-pyrrolidinyl)pyrimidine, GSK-J4, N-(4-{[6-(cyclobutylamino)-7H-purin-2-yl]amino}phenyl)-N-methylacetamide, 5-(4-methoxybenzyl)-2-(2-pyridinyl)-4,6-pyrimidinediamine, dasatinib, dimorpholinethiuram disulfide, FCCP, and PD184352.

In an embodiment, the compound for use is administered in an effective amount to a subject in need thereof.

In an embodiment, the compound for use of the present invention reduces the total kidney volume in a subject, preferably by at least 10%, at least 20% or at least 30%. In an embodiment, the compound for use of the present invention reduces the height-adjusted total kidney volume in a subject, preferably by at least 10%, at least 20% or at least 30%.

The term "Total kidney volume" or "TKV" is a measurement of total kidney volume. Total kidney volume may be determined by Magnetic Resonance Imaging (MRI), Computed Tomography (CT) scan, or ultrasound (US) imaging, and the volume calculated by a standard methodology, such as an ellipsoid volume equation (for ultrasound), or by quantitative stereology or boundary tracing (for CT/MRI).

The term "Height-adjusted total kidney volume" or "HtTKV" is a measure of total kidney volume per unit height. Patients with an HtTKV value ≥600 ml/m are predicted to develop stage 3 chronic kidney disease within 8 years.

In an embodiment, the invention relates to a combination product for use in the treatment of ADPKD, the combination product comprising as a first pharmaceutically active agent a compound of the present invention and as a second pharmaceutically active agent is tolvaptan. In an embodiment, the treatment of ADPKD comprises the co-administration of the compound of the present invention with tolvaptan. Co-administration can take place sequentially and/or simultaneously.

In an embodiment, the present invention relates to a method for treating autosomal polycystic kidney disease (ADPKD), the method comprising treating a subject in need thereof with a compound identified by the steps of:
(a) contacting a test compound with PKD1-deficient cells;
(b) determining cell viability of the PKD1-deficient cells; and
(c) confirming that cell viability of the PKD1-deficient cells is lower than that of isogenic control cells.

### Examples

The present invention is further illustrated by the following Examples and the Figures which represent preferred embodiments of the present invention:

### 1. Materials and Methods

### 1.1 Cell Culture

mIMCD-3 wild-type cells were obtained from ATCC (Manassas, VA, USA, CRL-2123; Lot 5064816). Cells were cultured with DMEM:F12 (Lonza, cat.no. BE12-719F), fetal bovine serum was added to a final concentration of 10% and penicillin/streptomycin (Sigma, P0781) to a final concentration of 1%. Subculturing of cells was performed twice a week in a 1:10 ratio. Cells were maintained in a humidified incubator at 37°C and 5% CO₂. Experiments were strictly performed with comparable and lowest possible passage numbers. The term "wild-type" is used for mIMCD-3 cells that were not edited by CRISPR-Cas9. The original cell line obtained from ATCC was generated from inner medullary collecting ducts of a SV-40 transgenic mouse.¹³

### 1.2 Generation of monoclonal PKD1 knockout cell lines with isogenic control cells

Details for the generation of monoclonal *PKD1* knockout (KO) cells can be found in more detail in prior publications.^{14,15} Briefly, a nickase-expressing plasmid pX335-U6-Chimeric-BB-CBh-hSpCas9n (Addgene, cat. no. 42335) with two different gRNAs was applied to induce two double strand breaks resulting in a *PKD1* null allele. Null alleles were confirmed by genomic sequencing, as well as complete absence of the respective mRNA and protein. Prior to genome editing, a monoclonal starting cell line was generated by a single cell sorting step. This monoclonal wildtype cell line allows for genome editing experiments with isogenic control cells thus reducing clonal variability and false-positive phenotypes in drug discovery as previously shown by our group.¹⁵

### 1.3 Generation of a monoclonal inducible PKD1 knockdown cell line

Monoclonal mIMCD-3 wildtype cells were transduced with SMARTvector Inducible Lentiviral shRNA (item number V3SM7671-231546250, Dharmacon Horizon) targeting mRNA of murine *PKD1.* Transduction was performed in semiconfluent 100 mm dishes with approximately 4,500,000 at time of transduction. Viral titer was adjusted to achieve a multiplicity of infection (MOI) of 0.3. Transduction media consisted of DMEM:F12 without fetal bovine serum or penicillin/streptomycin. Polybrene (Santa Cruz cat. no. 134220) was added to a final concentration of 10 µg/ml. Transduction media was removed after 24 hours and replaced by DMEM:F12 with 10% fetal bovine and 1% penicillin/streptomycin. After additional 24 hours cells were subcultured with selection media consisting of DMEM:F12 with 10% FBS and 1% penicillin/streptomycin and 6 µg/ml puromycin thus isolating cells with viral genome integration. Selection pressure was maintained for 5 days. After selection, doxycycline was added at a final concentration of 100 ng/ml. 48 hours after induction cells with a medium GFP intensity signal were single cell sorted with a BD FACS Aria III into 96-well plates (Greiner Bio one, flat bottom, transparent). Cells were maintained in a humidified incubator at 37°C and 5% CO₂, media was changed weekly. After 2.5 weeks, cell growth was monitored by visualization under an inverted microscope. Cells were expanded into 6-well plates and finally 100 mm dishes.

### 1.4 Cell seeding in 1536-well plate

One million mIMCD-3 cells were seeded 72 hours prior to the initiation of the experiment in cell culture flasks (TC Flask 175cm² SI Filter Cap, Thermo Fisher Scientific, cat#178883) prepared with 24 ml of DMEM:F12 (10% fetal bovine serum, 1% penicillin/streptomycin). After 72 hours cell density was checked with an inverted microscope (Zeiss Axiovert 40CFL). Cells were washed with 10 ml phosphate buffered saline (PBS). 3 ml of trypsin 0.25% (Gibco, cat. no 25200056) were added and incubated for 5-10 minutes. 10 ml DMEM:F12 with 10% FBS and 1% penicillin/streptomycin were added, cell detachment was facilitated by pipetting up and down multiple times. Cell density was determined with a Beckman Coulter Vi-cell XR with the software Vicell XR 2.04. Cell density was diluted to 400,000 cells/ml for each genotype. 5 µl of cell suspension per well (2,000 cells) were seeded into 1536-well plates (Greiner Bio one with circular basins at the edge of the plate) with a Thermo Scientific Multidrop combi dispenser using a Thermo Fisher small tube metal tip dispensing cassette (Cat no 24073295). Basins of 1536 well plates were filled with 25 µl (vertical basins) or 40 µl (horizontal basins) H₂O in order to prevent evaporation induced edge effects. Confluency was checked after 24 hours with an Evos M5000 imaging system (Invitrogen).

### 1.5 PKD1 knockdown induction

In experiments involving inducible *PKD1* knockdown cell lines, mRNA degradation was induced 7 days prior to cell seeding in 1536-well plates by incubating cells with 100 ng/ml doxycycline. Isogenic control cells were incubated in parallel in the absence of doxycycline. Knockdown efficiency was regularly tested either by western blot or indirectly by detection of GFP fluorescence after induction.

### 1.6 Compound transfer

Compounds for the primary screen had a source plate concentration of 5 mM. For the primary screen, 10 nl of compounds were added to destined 1536-well plate wells after 24 hours of incubation with an Echo 555 or Echo 655 liquid handler (Labcyte) resulting in a final concentration of 10 µM. Confirmation experiments were performed with a final compound concentration of 10 µM as well. For the secondary screen and validation experiments, source plates were generated with compounds in 1:2 dilutions allowing for the generation of dose-response-curves. 30 nl of compounds per well were transferred in secondary screen and validation experiments. Each plate screened contained two columns of negative (DMSO) and positive controls (digitonin, Calbiochem, Cat#300410), respectively. The volume of transferred DMSO was adjusted according to the transferred compound volume. Digitonin was used as a positive control in a final concentration of 180 µM (60 nl/ well). After compound transfer, plates were centrifuged (Multifuge 3 s-r, Heraeus) for 10 seconds (1,000 rpm). After centrifugation, plates were vortexed for 5 seconds (Teleshake 1536, Thermo Electron LED GmbH). Plates were stored in incubators at 37°C and 5% CO₂ for 72 hours. A maximum of two plates was stacked in the incubator. Source plates were sealed with an Agilent Technologies Plateloc heat sealer. Prior to compound transfer source plates were centrifuged (4,200 rpm, 1 min). Sealing was removed with a Brooks XPeel device.

### 1.7 Endpoint analysis

After 72 hours of compound incubation, plates were allowed to adapt to room temperature for 20 minutes. 2 µl media per well were removed with a BNX 1536 device (Bucher Biotec) followed by addition of 3 µl CellTiter-Glo 2.0 (Promega, Cat. #G9243) per well, added with a Multidrop combi dispenser (Thermo Scientific) using a small tube metal tip dispensing cassette (Thermo Fisher, Cat #24073295). Plates were briefly vortexed (Teleshake 1536, Thermo Electron LED GmbH). The luminescence signal was allowed to stabilize for 20 min. Luminescence was scanned with a BMG Labtech Pherastar FSX luminescence reader with a measurement interval time of 0.16 s per well. A 1536-well plate adapted aperture was used to minimize cross-talk. Data analysis was performed with GraphPad Prism (version 9). Dose- and genotype-dependent differences in viability were checked for statistical significance by performing a two-way ANOVA (α = 0.05). Difference in protein abundance analyzed by western blot were checked for statistical significance by performing a student's t-test (α = 0.05).

### 2. Results

### 2.1 Establishment of a luminescence-based viability assay for mIMCD-3 cells

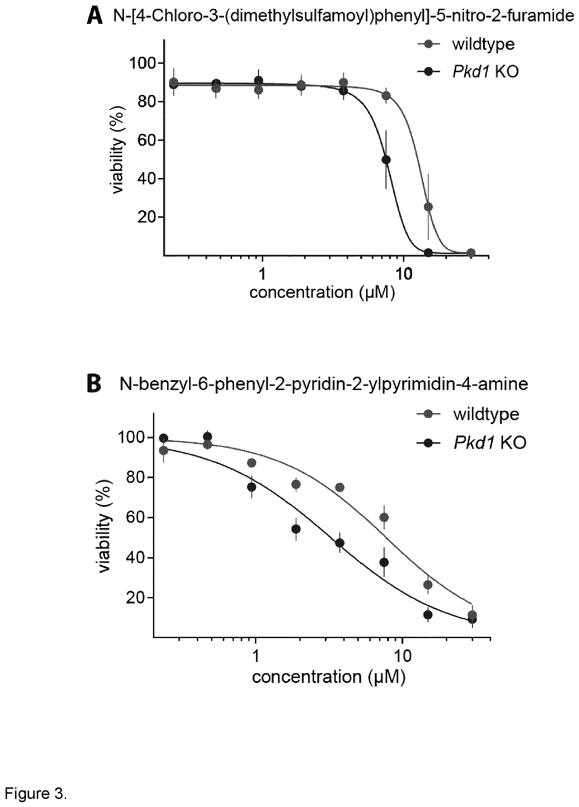

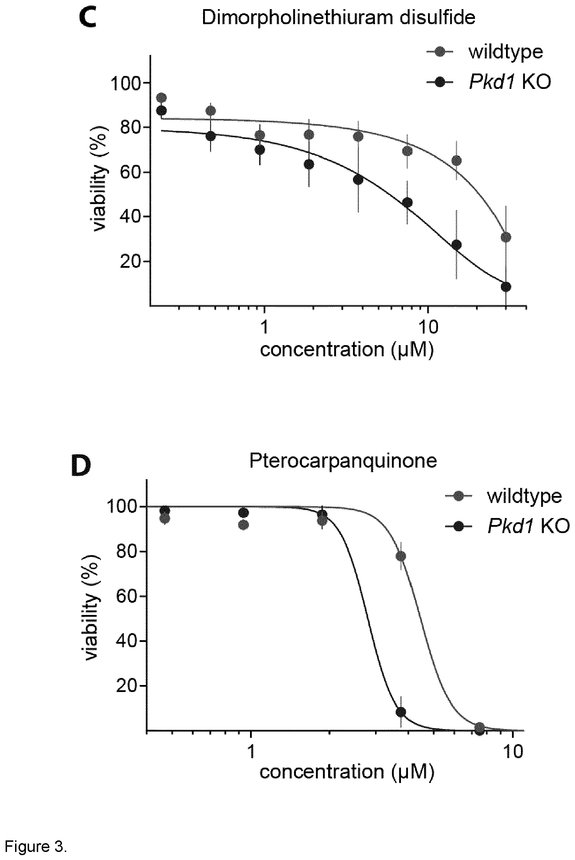

A synthetic lethality screen was developed based on differentiated confluent mIMCD-3 cells with apico-basal polarity as a model for polarized kidney tubular epithelial cells *in vivo.* A synthetic lethality approach on non-confluent cells would enrich hits that lead to proliferation inhibition in non-polarized cells instead of inducing cell death in a confluent polarized cyst epithelium, which is required to reduce cyst burden *in vivo.* An assay that favors the identification of compounds that induce selective death of homozygous *PKD1* mutant cells instead of inhibiting proliferation is more likely to identify compounds with the potential to reduce cystic burden in advanced stages of murine and/or human ADPKD. Since the aim was to identify compounds that selectively kill *PKD1* KO cells, multiple assays to monitor cell viability were tested and identified CellTiter-Glo 2.0 (Promega) as the most robust readout. CellTiter-Glo 2.0 is a luciferase-based assay that allows to indirectly interrogate cell viability by detecting an adenosin triphosphate (ATP)-dependent luminescence signal. Wildtype mlMCD-3 cells were seeded at different numbers in 384-well plates with an identical replicate plate. After 24 hours CellTiter Glo 2.0 luminescence signals in plate 1 were measured and correlated this signal with Hoechst-33342 DNA stain fluorescence signals in plate 2 (**Fig. 3****, A**). This experiment showed a strong correlation between mIMCD-3 cell number with the luminescence signal. In addition, it was tested whether the assay is suited to interrogate cell viability in mlMCD-3 cells after addition of cytotoxic compounds. To this end, dose-response curves were performed in wildtype mIMCD-3 cells using the cytotoxic agent digitonin and multiplexed Promega's CellTiter-Glo 2.0 with Promega's CellTox Green cytotoxicity assay. After disintegration of cell membranes this assay leads to fluorescence emission by fluorophore-deoxyribonucleic acid (DNA) interaction in cell nuclei thus allowing to quantify.

### 2.2 Establishing a protocol for high-throughput viability interrogation

To allow for more comprehensive drug testing, 1536-well plates were used. Seeding experiments demonstrated that 2,000 cells per well reliably lead to confluent mlMCD-3 monolayers after 24 hours incubation. A major problem in miniaturization is the occurrence of edge effects. In line with this frequently observed problem, considerable edge effects after 72h of compound incubation were observed (data not shown). Since it was known from previous experiments that lowering the compound incubation time results in less pronounced differential compounds effects, it was decided to use 1536-well plates that are surrounded by basins that can be filled with H₂O thus allowing to minimize edge effects caused by evaporation. Indeed, compound incubation in these plates was not associated with edge effects allowing to reliably incubate compounds for 72 hours. During optimization experiments, it was also found that the endpoint analysis with a BMG Pherastar luminescence reader was significantly improved by using a comparably low measurement interval time of 160 µs per well.

### 2.3 "SynLeth PKD" - a high-throughput synthetic lethality ADPKD assay

To test the concept of genotype-dependent synthetic lethality in mIMCD-3 cells, two distinct compound libraries were used. These libraries comprised publicly available compounds (SSEC_02, 49,280 compounds) as well as "Mode of Action" (MoA) annotated compounds (2,816 compounds). In summary, 52,096 compounds were included in the primary screen.

Since comprehensive small molecule screens are cost-intensive these projects mandate a streamlined screening strategy. Instead of simultaneously screening wildtype vs *PKD1* KO mlMCD-3 cells with dose-response curves, the drug screen was initiated with an exclusive interrogation of *PKD1* KO cell viability in a single compound concentration (10 µM). This strategy allowed to exclude compounds for comprehensive dose-response testing showing no or minor viability reduction effects thus minimizing total well number in the screening workflow. The small molecule screen was divided into five steps:
a) Primary screen
b) Confirmation
c) Secondary screen
d) Cluster analysis
e) Validation of hits from the secondary screen and cluster analysis

### 2.4 a) Primary screen

The primary screen was exclusively performed using a monoclonal *PKD1* KO cell line (clone #1). All compounds were tested with four biological replicates at 10 µM. As expected, the vast majority of compounds did not lead to viability reductions. However, 2,747 compounds were identified that resulted in a mean viability reduction of at least 25% and 953 compounds that led to a mean viability reduction of at least 50%. For hit labeling, the threshold was set to 25% mean viability reduction. Therefore, 2,747 compounds were included for subsequent confirmation analyses. In addition, the analysis of a Mode of Action library revealed 588 compounds showing a mean viability reduction of at least25%. In summary, 3,385 compounds were included for confirmation experiments.

### 2.5 b) Confirmation

3,385 compounds were identified in the primary screen as potential hits. These compounds were analyzed once again in the PKD1 KO mIMCD-3 cell line (clone #1). Each of these compounds was tested with three biological replicates. This confirmation analysis identified 1,824 false-positive hits thus reducing the total hits to 1,511 when applying a 25% threshold for viability reduction. These 1,511 compounds were labeled as true-positive hits and qualified to be analyzed in genotype-dependent dose response curves in a secondary screening step. The assay quality was excellent during the confirmation step.

### 2.6 c) Secondary Screen

To identify compounds with synthetic lethal properties depending on the *PKD1* genotype a secondary screen was conducted comparing *PKD1*-deficient cells and wildtype cells. The secondary screen included 1,511 compounds that were confirmed to reduce viability with an effect size of at least 25% in a monoclonal *PKD1* KO mIMCD-3 cell line (clone #1) at 10 µM. It was aimed to generate dose-response curves using this monoclonal *PKD1* knockout cell line while performing a simultaneous counter screen on the isogenic wildtype control cell line to identifiy compounds that kill *PKD1*-deficient cells more efficienty than wildtype cells. Hits were defined as compounds leading to selective reductions in cell viability in *PKD1* deficient cells compared to wildtype cells in at least two concentrations. It was found that the vast majority of compounds did not result in differential viability effects. However, according to hit criteria definition, 103 compounds were identified exhibiting differential viability effects. 30 compounds led to robust shifts in the IC₅₀ between wildtype and *PKD1* KO cell lines.

### 2.7 d) Cluster analysis

Based on the molecular structure of the hits, nine structural clusters were identified with potential differential effects on cell viability. Next, compounds with structural homologies to these nine clusters were selected and screened on *PKD1* KO clone #1, a second independent *PKD1 KO* clone (#2), and on the isogenic wildtype cells. 353 compounds were included in the cluster screening. 41 compounds showed stronger cytotoxicity in both *PKD1* deficient cell lines compared to wildtype cells (Data not shown).

### 2. 8 e) Validation of hits from the secondary screen and cluster analysis

These 41 compounds and the 30 compounds identified in the secondary screen were chosen to be validated more rigorously to exclude clonal artifacts in the genome-edited cell lines. To this end, it was decided to generate an inducible *PKD1* knockdown cell line. An inducible *PKD1* knockdown cell line allows to examine effects resulting from a sudden loss of polycystin-1 (PC1) and can also serve as a complementary approach to validate compounds identified with constitutive knockout cell lines.

All 71 compounds included in this validation step showed IC₅₀ shifts in at least one constitutive knockout cell line. To qualify for further assessment, it was decided to set a threshold of differential IC₅₀ shifts in both constitutive *PKD1* knockout clones as well as in the inducible *PKD1* knockdown cell line. This experiment identified 23 compounds with IC₅₀ shifts in all investigated cell lines

**Table 1: Viability of PKD1-deficient cells and PKD1 control cells**

| Number | Name | IC₅₀ PKD1-/- (clone 1) (µM) | IC₅₀ PKD1-/- (clone 2) (µM) | IC₅₀ wildtype (µM) |
|---|---|---|---|---|
| 1 | 1-methyl-3-[(Z)-(4-oxo-3-prop-2-enyl-1,3-thiazolidin-2-ylidene)amino]thiourea | 11,45 | 10,02 | >30 |
| 2 | 2-TEDC | 15,42 | 21,73 | 23,96 |
| 3 | 7-Chloro-N-(3-chloro-2-methylphenyl)-4-quinazolinamine | >30 | 14 | >30 |
| 4 | CCCP | 3,54 | 2,85 | 4,31 |
| 5 | 3-Methyl-2-[(E)-2-(5-nitro-2-furyl)vinyl]-4(3H)-quinazolinone | 2,39 | 2,64 | 4,01 |
| 6 | 3-(4-Hydroxyphenyl)-2-[(E)-2-(5-nitro-2-furyl)vinyl]-4(3H)-quinazolinone | 3,17 | 2,98 | 4,49 |
| 7 | N-(3-Chloro-2-methylphenyl)-6,7-dimethoxy-4-quinazolinamine | 16,98 | 12,13 | >30 |
| 8 | 5-Nitro-N-{2-[(2-pyridinylmethyl)carbamoyl]phenyl}-2-furamide | 19,40 | 19,36 | 24,48 |
| 9 | N-[3-Methoxy-5-(trifluoromethyl)phenyl]-5-nitro-2-furamide | 15,01 | 15.51 | 18,40 |
| 10 | N-benzyl-6-phenyl-2-pyridin-2-ylpyrimidin-4-amine | 3,24 | 6,03 | 7,73 |
| 11 | 6-(2-Fluorophenyl)-N-[2-(1H-indol-3-yl)ethyl]-2-(2-pyridinyl)-4-pyrimidinamine | 3,05 | 6,43 | 9,68 |
| 12 | N-[4-Chloro-3-(dimethylsulfamoyl)phenyl]-5-nitro-2-furamide | 7,09 | 8,51 | 11,21 |
| 13 | 4-Piperidin-1-yl-6-propan-2-yl-2-pyridin-2-ylpyrimidine | 6,75 | 18,68 | 28,69 |
| 14 | Refametinib | >30 | >30 | >30 |
| 15 | Pterocarpanquinone ((2S,10S)-3,12-dioxapentacyclo[11.8.0.02,10.04,9.015,20]henicosa-1(13),4,6,8,15,17,19-heptaene-14,21-dione) | 3,54 | 2,80 | 4.45 |
| 16 | 5-Chloro-4-methyl-2-(2-pyridinyl)-6-(1-pyrrolidinyl)pyrimidine | >30 | >30 | >30 |
| 17 | GSK-J4 | 3,41 | 6,08 | 7,69 |
| 18 | N-(4-{[6-(Cyclobutylamino)-7H-purin-2-yl]amino}phenyl)-N-methylacetamide | 3,07 | 2,53 | 5,93 |
| 19 | 5-(4-Methoxybenzyl)-2-(2-pyridinyl)-4,6-pyrimidinediamine | 20,02 | 24,90 | 36,53 |
| 20 | Dasatinib | 2,32 | 1,42 | 3,74 |
| 21 | Dimorpholinethiuram disulfide | 5,30 | 3,94 | 19,78 |
| 22 | FCCP | 2,89 | 2,42 | 4,12 |
| 23 | PD184352 | 6,21 | 7,96 | 10,27 |
| 24 | Mirdametinib | 80 | 21,78 | 2707 |
| 25 | Tak-733 | 16,85 | 9,51 | 41,94 |

Among these 23 compounds, 14 compounds were identified in the secondary screen and 9 compounds were identified in the cluster expansion analysis. Comparing the two hit rates between unbiased and rationalized screening shows that rationalized screening yields a 100-fold higher hit rate. Based on the fact that two compounds were MEK-inhibitors, we tested further MEK-inhibitors. Mirdametinib (no. 24) and Tak-733 (no. 25) showed strong effect sizes.

### Brief description of the Figures

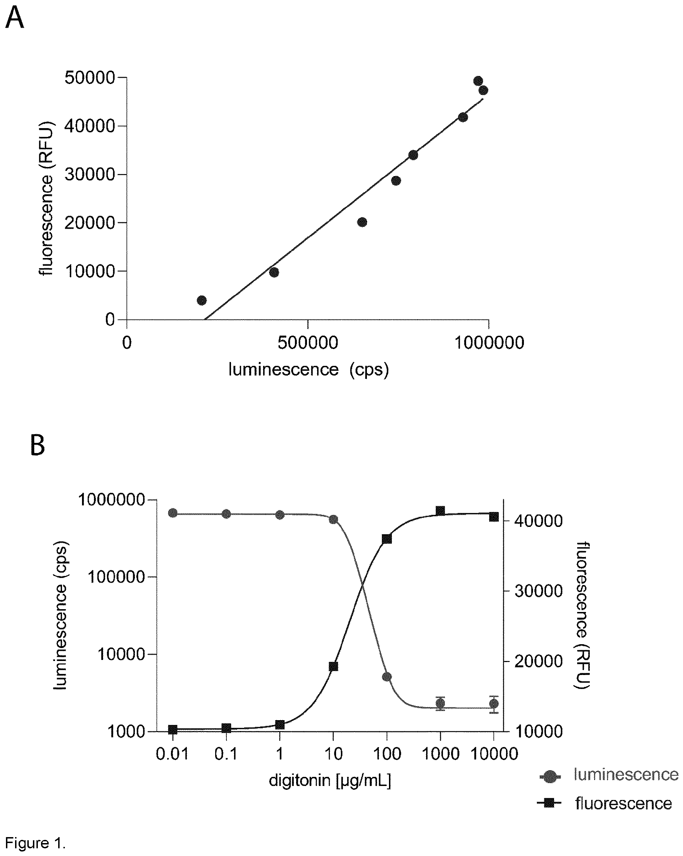

Figure 1 shows that cell viability can be determined with the CellTiter-Glo 2.0 assay. In Figure 3A, two 384-well plates (identical plate design) with eight different cell concentrations were incubated for 24 hours. Cells in plate 1 were incubated with CellTiter-Glo 2.0 reagent. Cells in plate 2 were incubated with Hoechst-33342 fluorophore. Luminescence and fluorescence were measured with a spectrophotometer for four technical replicates per cell concentration. Fluorescence and luminescence signals showed a strong correlation. Pearson correlation coefficient r=0,98; 95 % CI 0,88-0,99; r²=0,97; p <0,0001). In Figure 3B mIMCD-3 cells were cultured for 24 hours with multiple concentrations of digitonin. After 24 hours sequential measurements with CellTox green and CellTiter-Glo 2.0, respectively, were performed. Luminescence and fluorescence signals show an inverse relationship. Dots represent means of luminescence signal for four technical replicates for each concentration. Error bars represent SD. Squares represent means of fluorescence signal for four technical replicates for each concentration. Error bars represent SD. A dose-response curve was fitted with a sigmoidal 4-parameter-model.

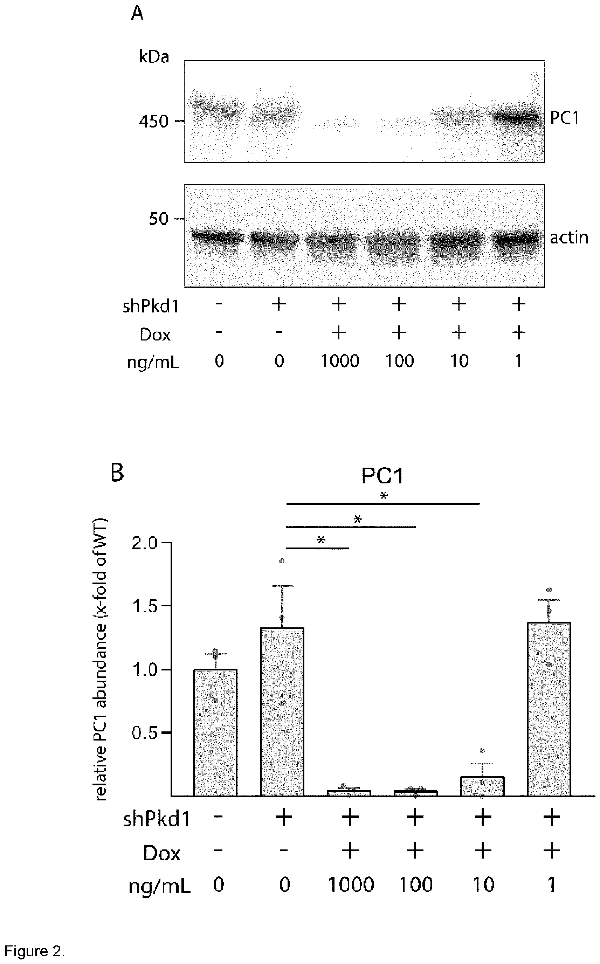

Figure 2 shows the evaluation of *PKD1* knockdown efficacy in stable shPKD1 mIMCD-3 cells. In Figure 2A, protein abundance of PC1 in cell lysates of mlMCD-3 wild-type and monoclonal shPKD1-transduced cell lines after incubation with and without doxycycline for seven days is shown. Doxycycline concentrations as indicated. In the absence of doxycycline the shPKD1 cell line shows PC1 levels comparable to wildtype cell line. Incubation with 1000 and 100 ng/mL doxycycline, respectively, leads to a strong decrease in protein levels of PC1. Lower concentrations of doxycycline did not induce a sufficient PC1 knockdown. Figure 2B depicts densitometry of three biological replicates confirms these findings. Bars illustrate mean relative protein abundance. Dots represent biological replicate. Error bars represent standard error of the mean.
Figure 3 shows dose response curves for exemplary compounds with increased selective cytotoxicity in *PKD1*-deficient cell lines compared to isogenic wildtype control cells. (**A-D**) Concentration-response curves for compounds with *Pkd1* genotype-dependent effects on cell viability. Compounds were tested in two independent knockout clones and in cells with doxycycline-inducible shRNA-mediated *Pkd1* knockdown. Experiments involving inducible knockdown experiments were conducted with a very low doxycycline concentration (100 ng/ml, induced) or with solvent (DMSO, control). Dots represent mean of four biological replicates. Error bars indicate SEM.

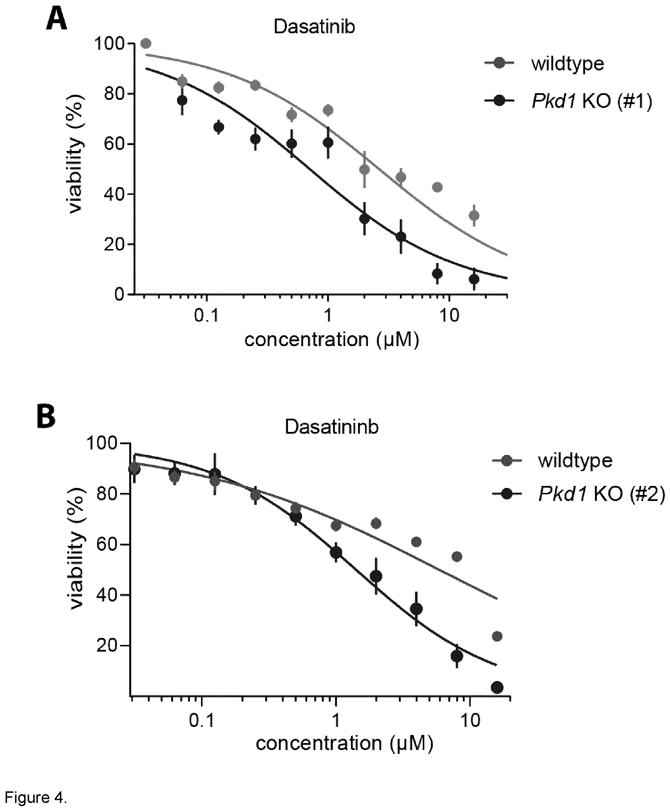

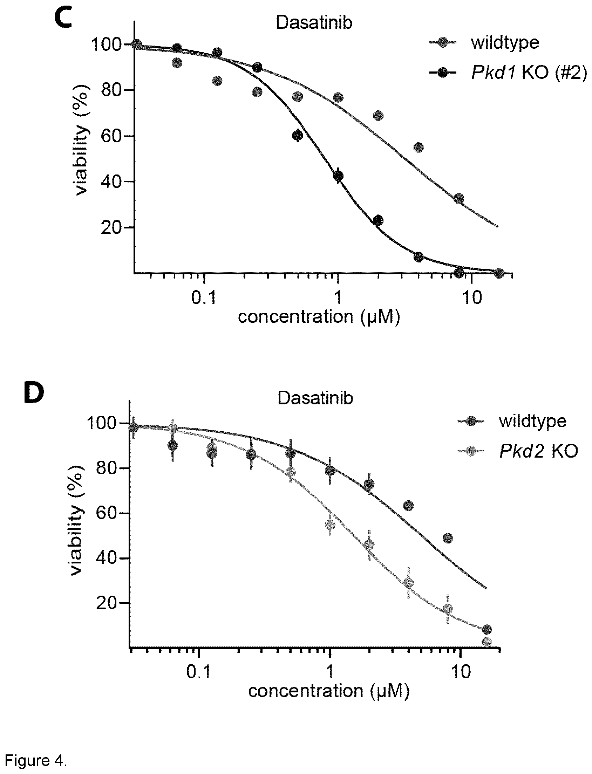

Figure 4 shows in (**A** and **B**) dose-response curves for constitutive *Pkd1*-deficient cell lines treated with dasatinib for 72 hours. (**C**) Dose-response curve for constitutive *Pkd1*-deficient cell line (clone #2) treated with dasatinib for 168 hours. (**D**) Dose-response curves for constitutive *Pkd2*-deficient cell line treated with dasatinib for 72 hours. (**A-D**) Dots represent mean of biological replicates (three or four biological replicates per comparison). Error bars represent SEM.

### REFERENCES

1 GBD Chronic Kidney Disease Collaboration. Global, regional, and national burden of chronic kidney disease, 1990-2017: a systematic analysis for the Global Burden of Disease Study 2017. Lancet 395, 709-733 (2020). https://doi.org/10.1016/S0140-6736(20)30045-3
2 Torres, V. E., Harris, P. C. & Pirson, Y. Autosomal dominant polycystic kidney disease. Lancet 369, 1287-1301 (2007). https://doi.org/10.1016/S0140-6736(07)60601-1
3 Collins, A. J. et al. US Renal Data System 2012 Annual Data Report. Am J Kidney Dis 61, A7, e1-476 (2013). https://doi.org/10.1053/j.ajkd.2012.11.031
4 Qian, F., Watnick, T. J., Onuchic, L. F. & Germino, G. G. The molecular basis of focal cyst formation in human autosomal dominant polycystic kidney disease type I. Cell 87, 979-987 (1996). https://doi.org/10.1016/s0092-8674(00)81793-6
5 Tan, A. Y. et al. Somatic Mutations in Renal Cyst Epithelium in Autosomal Dominant Polycystic Kidney Disease. J Am Soc Nephrol 29, 2139-2156 (2018). https://doi.org/10.1681/ASN.2017080878
6 Torres, V. E. et al. Tolvaptan in patients with autosomal dominant polycystic kidney disease. N Engl J Med 367, 2407-2418 (2012). https://doi.org/10.1056/NEJMoa1205511
7 O'Neil, N. J., Bailey, M. L. & Hieter, P. Synthetic lethality and cancer. Nat Rev Genet 18, 613-623 (2017). https://doi.org/10.1038/nrg.2017.47
8 Grantham, J. J., Chapman, A. B. & Torres, V. E. Volume progression in autosomal dominant polycystic kidney disease: the major factor determining clinical outcomes. Clin J Am Soc Nephrol 1, 148-157 (2006). https://doi.org/10.2215/CJN.00330705
9 Ashworth, A. & Lord, C. J. Synthetic lethal therapies for cancer: what's next after PARP inhibitors? Nat Rev Clin Oncol 15, 564-576 (2018). https://doi.org/10.1038/s41571-018-0055-6
10 Baigent, C. et al. Challenges in conducting clinical trials in nephrology: conclusions from a Kidney Disease-Improving Global Outcomes (KDIGO) Controversies Conference. Kidney Int 92, 297-305 (2017). https://doi.org/10.1016/j.kint.2017.04.019
11 Grantham, J. J. et al. Volume progression in polycystic kidney disease. N Engl J Med 354, 2122-2130 (2006). https://doi.org/10.1056/NEJMoa054341
12 Grantham, J. J. & Torres, V. E. The importance of total kidney volume in evaluating progression of polycystic kidney disease. Nat Rev Nephrol 12, 667-677 (2016). https://doi.org/10.1038/nrneph.2016.135
13 Rauchman, M. I., Nigam, S. K., Delpire, E. & Gullans, S. R. An osmotically tolerant inner medullary collecting duct cell line from an SV40 transgenic mouse. Am J Physiol 265, F416-424 (1993). https://doi.org/10.1152/ajprenal.1993.265.3.F416
14 Hofherr, A. et al. Efficient genome editing of differentiated renal epithelial cells. Pflugers Arch 469, 303-311 (2017). https://doi.org/10.1007/s00424-016-1924-4
15 Westermann, L. et al. Wildtype heterogeneity contributes to clonal variability in genome edited cells. Sci Rep 12, 18211 (2022). https://doi.org/10.1038/s41598-022-22885-8

## Claims

1. A process for identifying a compound useful for treating autosomal dominant polycystic kidney disease (ADPKD), the process comprising:
(a) contacting a test compound with PKD1-deficient and/or PKD2-deficient cells;
(b) determining cell viability of the PKD1-deficient and/or PKD2-deficient cells; and
(c) identifying useful compounds by confirming that cell viability of the PKD1-deficient and/or PKD2-deficient cells is lower than that of isogenic control cells.

2. The process according to claim 1, wherein cell viability is measured by a cell viability assay, preferably a cell counting assay, a terminal deoxynucleotidyl transferase nick end labeling assay, an immunohistochemical assay, or a ATP-dependent assay.

3. The process according to any of claims 1 or 2, wherein the PKD1-deficient and/or PKD2-deficient cells are homozygous cells, preferably PKD1 and/or PKD2 knockout cells.

4. The process according to any of claims 1 to 3, wherein the PKD1-deficient and/or PKD2-deficient cells are obtainable by genome editing of mIMCD-3 cells.

5. The process according to any of claims 1 to 4, wherein the PKD1-deficient and/or PKD2-deficient cells are obtainable by using a CR!SPR-Cas nickase system, preferably a CRISPR-Cas9 system.

6. The process according to any of claims 1 to 5, wherein the compound useful for treating ADPKD has a ratio of IC₅₀ for PKD1-deficient cells to IC₅₀ for isogenic control cells of less than 0.85, less than 0.90, less than 0.85 or less than 0.80.

7. The process according to any one of claims 1 to 6, wherein contacting in step (a) takes place at about 37°C for at least 12 hours, at least 24 hours, at least 36 hours, at least 48 hours, or at least 60 hours.

8. The process according to any of claims 1 to 7, wherein the process is a high-throughput screening assay.

9. The process according to any of claims 1 to 8, wherein the contacting step takes place on well plates, preferably well plates that comprise basins that contain water.

10. Compound for use in the treatment of autosomal dominant polycystic kidney disease (ADPKD),
wherein the compound is selected from a compound formula (I): 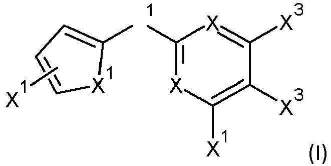 wherein
R¹ is selected from nitro, cyano, halo, alkoxy, alkyl and carboxy,
X¹ is selected from NH, N(alkyl), and O,
L¹ is a linker group selected from C₁-C₆ alkylene, C₂-C₆ alkenylene, NHC(O), C(O)NH, S(O)₂NH, NHS(O)₂, NHC(O)NH, and S(O)₂,
X² is selected from CR^{0a}, and N,
X³ is selected from CR^{0c}, and N,
R^{0a}, R^{0c}, and R¹ are independently selected from hydroxy, halo, hydrogen, alkyl, haloalkyl, alkoxy, haloalkoxy, sulfamoyl, sulfamoylalkyl, N,N-(dialkyl)-sulfamoyl, and N-alkylsulfamoyl,
R² and R³ form, together with the carbon atoms to which they are attached, an optionally substituted 5- or 6-membered aromatic group, or R² and R³ are independently selected from halo, hydrogen, alkyl, haloalkyl, alkoxy, haloalkoxy, sulfamoyl, sulfamoylalkyl, N,N-(dialkyl)-sulfamoyl, and N-alkylsulfamoyl;
a compound of formula (II): 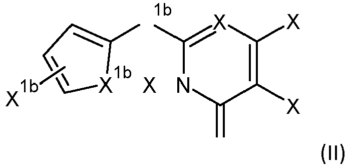 wherein
R^{1b} is selected from nitro, cyano, halo, alkoxy, alkyl and carboxy,
X^{1b} is selected from NH, N(alkyl), and O,
L^{1b} is a linker group selected from C₁-C₆ alkylene, C₂-C₆ alkenylene, NHC(O), C(O)NH, S(O)₂NH, NHS(O)₂, NHC(O)NH, and S(O)₂,
X^{2b} is CR^{1b} or N,
R^{0b} and R^{1b} are independently selected from optionally substituted 5- or 6-membered aromatic group, hydroxy, halo, hydrogen, alkyl, haloalkyl, alkoxy, haloalkoxy, sulfamoyl, sulfamoylalkyl, N,N-(dialkyl)-sulfamoyl, and N-alkylsulfamoyl,
R^{2b} and R^{3b} form, together with the carbon atoms to which they are attached, an optionally substituted 5- or 6-membered aromatic group, or R^{2b} and R^{3b} are independently selected from halo, hydrogen, alkyl, haloalkyl, alkoxy, haloalkoxy, sulfamoyl, sulfamoylalkyl, N,N-(dialkyl)-sulfamoyl, and N-alkylsulfamoyl;
a compound of formula (III): 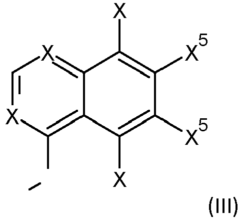 wherein
X⁴ and X⁵ are independently selected from CH and N,
L² is NH, O, or S(O)₂,
L³ is an optionally substituted 5- or 6-membered aromatic group, and
R⁴ to R⁷ are independently selected from H, halo, nitro, cyano, optionally substituted alkoxy, alkyl, and haloalkyl;
MEK inhibitors, 1-methyl-3-[(Z)-(4-oxo-3-prop-2-enyl-1,3-thiazolidin-2-ylidene)amino]thiourea, 2-TEDC, CCCP, 5-nitro-N-{2-[(2-pyridinylmethyl)carbamoyl]phenyl}-2-furamide, N-benzyl-6-phenyl-2-pyridin-2-ylpyrimidin-4-amine, 6-(2-fluorophenyl)-N-[2-(1H-indol-3-yl)ethyl]-2-(2-pyridinyl)-4-pyrimidinamine, 4-piperidin-1-yl-6-propan-2-yl-2-pyridin-2-ylpyrimidine, pterocarpanquinone, 5-chloro-4-methyl-2-(2-pyridinyl)-6-(1-pyrrolidinyl)pyrimidine, GSK-J4, N-(4-{[6-(cyclobutylamino)-7H-purin-2-yl]amino}phenyl)-N-methylacetamide, 5-(4-methoxybenzyl)-2-(2-pyridinyl)-4,6-pyrimidinediamine, dasatinib, dimorpholinethiuram disulfide, FCCP, and PD184352; as well as pharmaceutically acceptable salts, solvates, polymorphs, enantiomers, and tautomers thereof.

11. The compound for use according to claim 10, wherein
X⁴ and X⁵ are N,
L² is NH,
L³ is an optionally substituted phenyl group, preferably a disubstituted phenyl group, and
R⁴ to R⁷ are independently selected from H, halo, and alkoxy.

12. The compound for use according to claim 10 or 11, wherein
R¹ is selected from nitro, cyano, and halo;
X¹ is selected from NH, N(alkyl), and O;
L¹ is a linker group selected from NHC(O), C(O)NH, S(O)₂NH, NHS(O)₂, NHC(O)NH, and S(O)₂,
X² is CR^{0a},
X³ is selected from CR^{0c},
R^{0a}, R^{0c}, and R¹ are independently selected from hydroxy, halo, hydrogen, alkyl, haloalkyl, alkoxy, haloalkoxy, sulfamoyl, sulfamoylalkyl, N,N-(dialkyl)-sulfamoyl, and N-alkylsulfamoyl, and
R² and R³ are independently selected from halo, hydrogen, alkyl, haloalkyl, alkoxy, haloalkoxy, sulfamoyl, sulfamoylalkyl, N,N-(dialkyl)-sulfamoyl, and N-alkylsulfamoyl.

13. The compound for use according to any one of claims 10 to 12, wherein
R^{1b} is selected from nitro, cyano, and halo,
X^{1b} is selected from NH, N(alkyl), and O;
L^{1b} is a linker group selected from C₁-C₆ alkylene and C₂-C₆ alkenylene,
X^{2b} is N,
R^{0b} is selected from optionally substituted 5- or 6-membered aromatic group and alkyl, and
R^{2b} and R^{3b} form, together with the carbon atoms to which they are attached, an optionally substituted 5- or 6-membered aromatic group.

14. The compound for use according to any one of claims 10 to 13, wherein
the compound of formula (I) is N-[3-Methoxy-5-(trifluoromethyl)phenyl]-5-nitro-2-furamide or N-[4-Chloro-3-(dimethylsulfamoyl)phenyl]-5-nitro-2-furamide;
the compound of formula (II) is 3-(4-hydroxyphenyl)-2-[(E)-2-(5-nitrofuran-2-yl)ethenyl]quinazolin-4-one or 3-methyl-2-[(E)-2-(5-nitrofuran-2-yl)ethenyl]quinazolin-4-one; and
the compound of formula (III) is 7-chloro-N-(3-chloro-2-methylphenyl)-4-quinazolinamine, N-(3-chloro-2-methylphenyl)-6,7-dimethoxy-4-quinazolinamine, or N-(3-chloro-2-methylphenyl)-4-quinazolinamine.

15. The compound for use according to any one of claims 10 to 14, wherein the MEK inhibitor is selected from refametinib, mirdametinib, and Tak-733.
